# EUROPEAN PATENT SPECIFICATION

(11) **EP 1 800 604 B1**
(45) Date of publication and mention of the grant of the patent: **09.09.2009**
(21) Application number: 05790538.2
(22) Date of filing: 05.10.2005
(51) Int. Cl.: A61B 8/08

(54) **ULTRASONIC DIAGNOSIS DEVICE**
ULTRASCHALL-DIAGNOSEGERÄT
DISPOSITIF DE DIAGNOSTIC ULTRASONORE

(30) Priority: 08.10.2004 JP 2004296207
(43) Date of publication of application: 27.06.2007
(73) Proprietor: Hitachi Medical Corporation, Chiyoda-ku Tokyo (JP)
(72) Inventor: WAKI, Koji c/o HITACHI MEDICAL CORPORATION, Tokyo, 101-0021 (JP)
(74) Representative: Strehl Schübel-Hopf & Partner
(86) International application number: PCT/JP2005/018413
(87) International publication number: WO 2006/040967

(56) References cited:
- EP-A- 1 762 180
- WO-A-01/39668
- WO-A-20/04039262
- JP-A- 3 139 338
- JP-A- 2004 261 198
- JP-A- 2004 267 464
- JP-A- 2004 351 062
- JP-A- 2005 066 041
- US-B1- 6 359 367

## Description

### Technical Field

The present invention relates to an ultrasound diagnostic apparatus for displaying an ultrasound image of the diagnostic region of a subject to be examined using ultrasonic waves, particularly to the ultrasound diagnostic apparatus for displaying a strain/elasticity modulus image.

### Technical Background

An ultrasound diagnostic apparatus measures ultrasound reflectivity of biomedical tissues using ultrasonic waves, converts the measurement into luminance, and displays it as a reflectivity tomographic image. Recently, in an ultrasound diagnostic apparatus, displaying a strain or elastic modulus of biomedical tissues as an image has been implemented by measuring the strain through performing image correlation or performing spatial differentiation on the shifting distance, for example, the displacement of the tissues, or by measuring the elastic modulus of the tissues through giving pressure change to them as tissue diagnosis. This image is imparted with hue information such as red, blue and other hues according to the strain amount or elastic modulus of the biomedical tissues. In an ultrasound diagnostic apparatus, it is set so that the extent or size of a tumor can be easily diagnosed by imparting colors mainly to the hard portions of the biomedical tissues.

For example, as disclosed in JP-A-2003-225239, a compression pressure force is detected using a pressure sensor, an elastic image is constructed using the detected information by implementing hue modulation on the strain or elasticity of the tissues, and these images are superimposed on the black and white tomographic image (B-image) to be displayed. In other words, the elasticity of tissues is imaged and displayed recognizable as a relative strain or hardness of the tissues.
While the elasticity of tissues is for imaging the strain of tissues upon being pressed, the degree of strain differs in each target portion, thus there is a suitable degree of compression with respect to each target portion. Therefore, giving inadequate compression would result in constructing images having artifacts and could lead to a misdiagnosis.
An ultrasound diagnostic apparatus with which the invention has the features recited in the pre-characterising first part of claim 1 in common is disclosed in Figure 11 of WO 01/39668 A1. The apparatus of that prior art displays an elasticity image obtained from pressure sensors, in parallel with an ultrasonic image obtained from an ultrasonic probe. The output from the pressure sensors may also signal to an operator whether the amount of pressure applied by the ultrasonic probe to the imaged tissue is adequate.
EP 1762180 A1 which is prior art under Article 54(3) EPC discloses an ultrasound diagnostic apparatus which can display an elasticity image calculated from the pressure detected between an ultrasonic probe and the tissue, and from the tissue displacement resulting from the pressure and measured in an ultrasonic image. A display shows the applied pressure, the elasticity image and the ultrasonic image.

### Summary of the invention:

It is an object of the invention to provide an ultrasound diagnostic apparatus capable to display a strain elasticity image and to clearly display the pressure conditions when the ultrasonic probe is pressed to biomedical tissue.
This object is solved by the apparatus set forth in claim 1. The dependent claims relate to preferred embodiments of the invention.

An ultrasound diagnostic apparatus of an embodiment comprises:
an ultrasonic probe;
an ultrasonic transmitting/receiving unit for causing the ultrasonic probe to perform transmission and reception of ultrasonic waves between the probe and a subject or a phantom;
signal processing means for processing signals detected by the ultrasonic probe to construct a strain elasticity image; and
display means for displaying the strain elasticity image;
characterized in having pressure detecting means for detecting pressure at a plurality of positions between the subject or the phantom and the ultrasonic probe,
wherein pressure condition in the plurality of positions are displayed on the display means.

The ultrasonic probe is provided with a pressure-aid coupler in which a plurality of pressure detecting means are mounted. Also, when the pressure is insufficient in any direction of the plurality of pressure detecting means, the display means displays the direction that lacks the pressure, displays the pressure information corresponding to the arrangement of the pressure detecting means with contrasting density or arrow, and displays the same information in graph form. The display means further displays the pressure graph at the position corresponding to the pressure detecting means on the strain elasticity image, displays respectively the indicator arrows for rotating the probe centering around the major axis and minor axis of the probe based on the pressure condition of the probe, displays in parallel the strain elasticity image obtained in real time and the previously imaged elasticity model image, and displays the obtained pressure information in graph form as time advances.

Furthermore, the ultrasound diagnostic apparatus comprises detecting means for detecting the degree of coincidence between the strain elasticity image obtained in real time and the elasticity model image by superimposing the strain-elasticity image obtained in real time and the previously imaged elasticity model image. The pressure detecting means is a cMUT element, and detects the pressure by variance of electrostatic capacity.

### Brief Description of the Diagrams

Fig. 1 is a block diagram for carrying out the present invention.
Fig. 2 is a diagram showing an example of the display image of the present invention.
Fig. 3 is an explanatory diagram for detecting the coincidence of images in the present invention.
Fig. 4 is a diagram showing the pressure graph display of the present invention.
Fig. 5 is a configuration diagram of the probe in the present invention.
Fig. 6 is a diagram showing the configuration of the pressure measurement in the present invention.
Fig. 7 is a diagram showing the display of pressure distribution in the present invention.
Fig. 8 is a diagram showing the examples of the display image in the present invention.
Fig. 9 is a diagram showing an example of the training phantom simulating the living body.
Fig. 10 is a diagram showing an example of manufacturing method of the training phantom in Fig. 9.
Fig. 11 is a diagram showing an example of production method of the training phantom in Fig. 9.

### Best Mode for Carrying Out the Invention

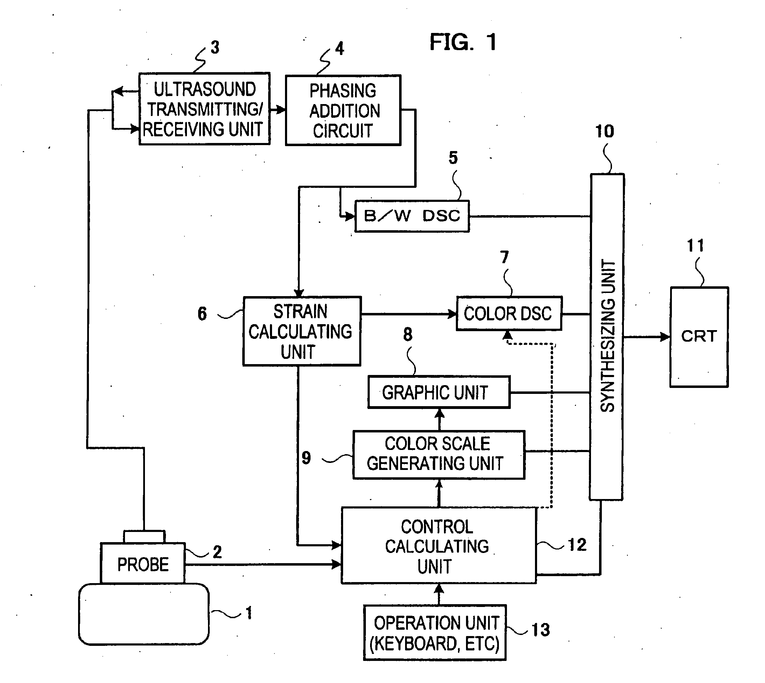

Hereinafter, an embodiment of the present invention will be described based on the attached diagrams. Fig. 1 is a diagram showing the schematic configuration of the ultrasound diagnostic apparatus relating to the present embodiment.

The ultrasound diagnostic apparatus is configured comprising:
probe 2 having devices such as a pressure sensor for applying on the training phantom or subject 1;
ultrasound transmitting/receiving unit 3 for repeatedly transmitting/receiving ultrasonic waves to/from the training phantom or subject 1 via probe 2 at certain time intervals;
phasing addition circuit 4 for performing phasing addition on the received reflection echo and creating the RF signal data;
tomographic image constructing unit 5 for constructing a grayscale tomographic image, for example, the black and white tomographic,image of the training phantom or subject 1 based on RF signal data;
strain calculating unit 6 for obtaining elasticity data by measuring displacement of the training phantom or biomedical tissues of subject 1 based on RF signal data from phasing addition circuit 4;
elasticity image constructing unit 7 for constructing a color elasticity image;
graphic unit 8 for drawing images using signals other than ultrasound signals;
color scale generating unit 9;
synthesizing unit 10 for synthesizing the black and white tomographic image, color elasticity image, graphic and color scale on the same image;
image displayer 11 for displaying the synthesized image;
control calculating unit 12 for controlling the respective components; and
keyboard 13 as an interface for the respective settings.

Probe 2 has a plurality of transducers and is provided with a function that transmits/receives ultrasonic waves via transducer to/from the training phantom or subject 1 by electronically executing beam scanning. Ultrasound transmitting/receiving unit 3 has a function for creating the transmission pulse for generating ultrasonic waves by activating probe 2, as well as setting the convergent point of the transmitted ultrasonic waves at a certain depth upon transmission. Also, upon reception, ultrasound transmitting/receiving unit 3 generates RF signals that are reception signals by amplifying the reflected echo signals received by probe 2 at predetermined gain. Phasing addition circuit 4 generates RF signal data by inputting the amplified RF signals and performing phasing control on them, and forming the converged ultrasound beams corresponding to the plurality of convergent points.

Tomographic image constructing unit (B/W DSC) 5 is configured including the signal processing unit/black and white scan converter. Here, it is for acquiring the tomographic image data by inputting the RF signal data from phasing addition circuit 4 and performing the signal processing such as log compression, detection, edge enhancement and filtering. Also, the black and white scan converter is configured including devices such as an A/D converter for converting the tomographic image data from the signal processing unit into digital signals, a frame memory for storing the converted plurality of tomographic image data in time series and a controller. Tomographic image constructing unit 5 is for obtaining the tomographic frame data of the training phantom or subject 1 and is stored in the black and white scan converter or frame memory as 1 image, and reading out the obtained tomographic image frame data using TV synchronism.

Strain calculating unit 6 is configured including the RF signal selecting unit and the displacement-calculating unit and placed in the latter stage of phasing addition circuit 4 being diverged from the circuit. The RF-signal selecting unit is configured including a frame memory and a selecting unit. This RF-signal selecting unit is for storing the plurality of RF signal data from phasing addition circuit 4 in the frame memory, and selecting a pair of, that is two, RF signal frame data by the selecting unit from the stored RF signal frame data group. For example, the RF signals selecting unit sequentially stores, from phasing addition circuit 4, the RF signal data being created based on the time series that is the frame rate of the image, selects RF signal frame data (N) which is stored at the moment as a first data by the selecting unit according to the command from control calculating unit 12, and at the same time selects one RF signal frame data (X) out of the RF signal frame data group (N-1, N-2, N-3... N-M) that had been stored in the memory in the past. Here, N, M and X are the index numbers for indicating RF signals frame data, and should be a natural number.

The displacement-calculating unit is for acquiring the displacement of biomedical tissues from a pair of RF signal frame data. For example, the displacement calculating unit obtains 1-dimensional or 2-dimensional displacement distribution relating to displacement and shifting vector of biomedical tissues corresponding to the respective points of a tomographic image that is the direction and amount of the displacement, from a pair of data selected from the RF signals selecting unit that is RF signals frame data (N) and RF signals frame data (X), by carrying out 1-dimensional or 2-dimensional correlation process. Here, the block-matching method is used for the detection of the shifting vector.

The block-matching method is a process to divide an image into blocks, for example, formed by NxN pixels, focus attention on a block within the region of interest, search for the block that is the most approximated to the focused block from the previous frame, and determine the sample value by the predictive encoding that is the difference referring to the searched block.

Data of strain is calculated by performing spatial differentiation on the shifting distance, for example, the displacement of biomedical tissues. Also, data of elasticity modulus is calculated by dividing the change of pressure by the change of shifting distance. For example, when the displacement measured by the displacement calculating unit is set as ΔL and the pressure measured by the pressure-measuring unit (not shown in the diagram) is set as ΔP, since strain (S) can be acquired by performing the special differentiation on ΔL, the strain can be obtained using the formula, S = ΔL / Δ X.

Moreover, Young's modulus "Ym" of elasticity data can be calculated by the formula, Ym = (ΔP) / (ΔL/L). Because the elasticity of biomedical tissues corresponding to the respective points of a tomographic image can be acquired by this Young's modulus, 2-dimensional elastic image data can be obtained continuously. In addition, Young's modulus is the ratio with respect to the simple tensil stress added to an object and the distortion caused parallel to the tension.

Elastic image constructing unit (color DSC) 7 is configured including the elasticity data processing unit and the color scan converter. The elasticity data processing unit is for storing elasticity frame data outputted in time series from strain calculating unit 6 to the frame memory, and executing an image processing on the stored frame data by the image-processing unit according to the command of control calculating unit 12.

The color scan converter is for converting elasticity frame data into hue information based on the data from the elasticity data processing unit. It converts elasticity frame data into three primary colors of light that are red (R), green (G) and blue (B). For example, at the same time as converting the elasticity data with large strain into red code, the elasticity data with small strain is converted into blue code. The gradation sequence of red (R), green (G) and blue (B) is 256 gradations, and data value "255" indicates that it will be displayed in large luminance while data value "0" means that it will not be displayed at all. This color scan converter is connected to operation unit 13 formed by devices such as a keyboard, and is set so that factors such as hue of the elastic image is controlled by this operation unit 13. Also, mounting the pressure sensor to probe 2 makes it possible to measure the pressure applied upon pressing probe 2 on the training phantom or subject 1, and the pressure information thus measured can also be downloaded and measured by control calculating unit 12 and displayed by display unit 11.

Synthesizing unit 10 is configured comprising a frame memory, image-processing unit and image-selecting unit. Here, the frame memory is for storing data from tomographic image constructing unit 5, elastic image constructing unit 7 and graphic unit 8. Also, the image-processing unit is for adding the tomographic image data and elastic image data stored in the frame memory at the rate set in advance and synthesizing them, according to the command of the control unit. The luminance information and hue information of the respective pixel of the synthesized image are the result of adding the respective information on the black and white tomographic image and the colored elastic image at the setting proportion. Further, the image-selecting unit is for selecting the image to display on image displayer 11 out of the tomographic image data and elastic image data in the frame memory and the synthesized image data in the image-processing unit according to the command of the control unit.

Control calculating unit 12 downloads the black and white data, strain data obtained from the strain calculating unit, pressure data acquired from the pressure sensor mounted in tip of the probe and weighs these data and the data stored in the data base within, and is for displaying the appropriate pressure condition or for providing guidance to a user by sonant. These embodiments will be described below using concrete examples.

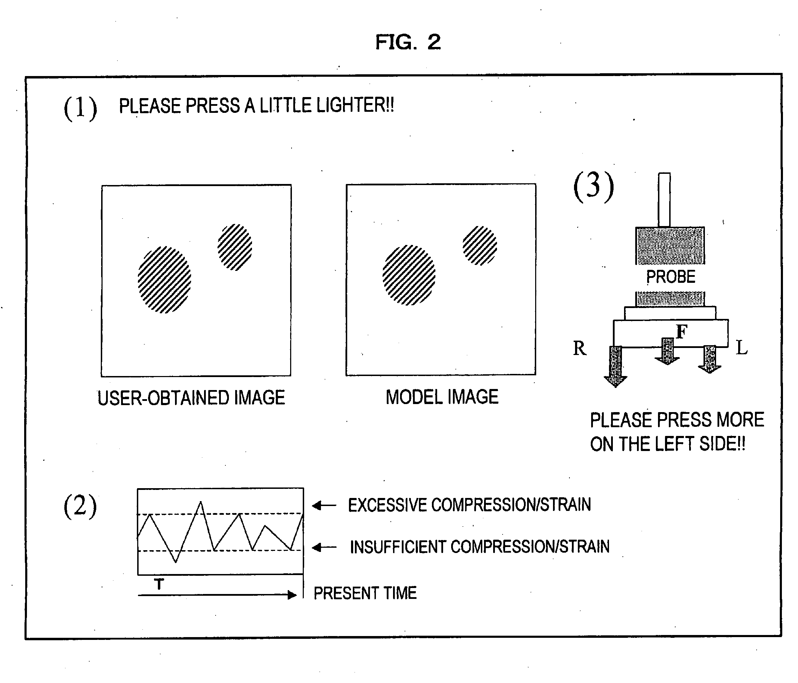

Fig. 2 is a diagram showing an example of the display by an ultrasound diagnostic apparatus relating to the present embodiment. On image displayer 11, a grayscale tomographic image (black and white tomographic image data) of the training phantom or subject 1 outputted from tomographic image constructing unit 5 and a tissue elastic image constructed by the elastic image constructing unit 7 based on the strain (elasticity) data are displayed in Fig. 2 (1). The tissue elastic images to be displayed are an image wherein a user uses the training phantom simulating the living body or subject 1 (user-obtianed image) and an image of an example created by graphic unit 8 which is imaged and stored in advance (model image), and these images are displayed in parallel on the screen. Therefore, the user can easily make judgment whether the compressed condition at the moment is adequate or not by visually comparing the user-obtained image being pressed at the moment and the model image. The plural types of example image for a training phantom or subject 1 are provided with respect to each target region. The ultrasound diagnostic apparatus is provided with a table corresponding to the ID of the training phantom or subject 1 with respect to each target region. Upon the training mode being selected, display of the body mark of the target region or the training for displaying the suitable compression method for each target region become possible by inputting information such as ID of the training phantom or subject 1 to control calculating unit 12 via operation unit 13.

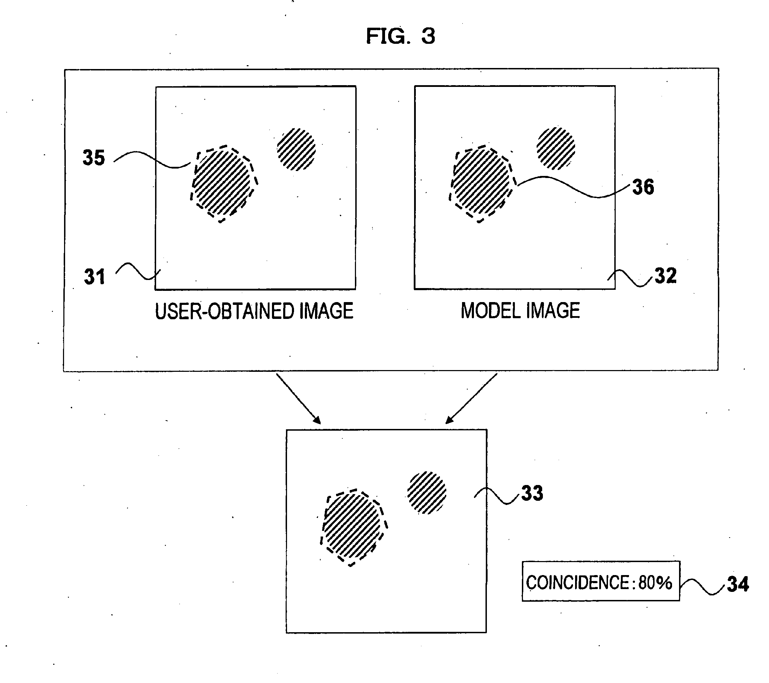

Also, as shown in Fig. 3, the coincidence of user-obtained image 31 and model image 32 is detected by superimposing them, and the detected coincidence is displayed. Whether the degree of compression is best suited or not is recognized by the user through this procedure.
In concrete terms, model image 32 and user-obtained image 31 are superimposed by synthesizing unit 10. In order to make the image position of model image 32 coincide with user-obtained image 31, the position of probe 2 in both images need be made to coincide.

If the comparison target is the training phantom, the pattern of the application surface of probe 2 should be marked on the phantom. Upon obtaining the model image 32 and user-obtained image 31, the probe 2 can be applied on the same marking position. Also, if the comparison target is a subject, the above-mentioned marking cannot be carried out, thus the 3-dimensional position of probe 2 upon obtaining model image 32 and use-obtained image 31 needs to be made coincide. Concretely, either an oscillator or a receiver as disclosed in Japanese Patent 1998-151131A is mounted in probe 2, and it induces control calculating unit 12 to recognize what part probe 2 is positioned on subject 1. Using this method, the position of probe 2 on the image stored in advance to be the model image is recognized by control calculating unit 12, the 3-dimensional position is displayed on image displayer 11, and the user is prompted by marks such as an arrow to make the probe 2 coincide with the displayed position.

Also, control-calculating unit 12 detects the coincidence of user-obtained image 31 with model image 32. As for the coincidence detection method, blue pattern 36 shown in the malignant region on model image 32 is registered as a binarization image, and the weighted center of blue pattern 35 on user-obtained image 31 is shifted and overlapped to coincide with the weighted center of blue pattern 36 on model image 32. Based on superimposed image 33, the area and the pixel count of the respective parts that are not overlapped are obtained and whether the pressure is appropriate or not is evaluated according to the obtained size and the pixel count of the areas that are not overlapped. If all of the respective blue patterns 35 and 36 coincide, it is displayed as "coincidence 100%" on image displayer 11. Also, if blue pattern 35 of user-obtained image 31 deviates 20% from blue pattern 36 of model image 32, it is displayed as "coincidence 80%" in display box 34 on image displayer 11. While the blue pattern is taken as an example here, any color may be used, and the red pattern for benignancy may also be the basis. Also, while the coincidence is acquired on the basis of the weighted center of blue patterns 35 and 36 and superimposing the images, only the size of the area of blue patterns 35 and 36 may be compared and evaluated.

Furthermore, in the case the area of blue pattern 35 of user-obtained image 31 is bigger compared to blue pattern 36 of model image 32, an attention message relating to compression such as "Please press a little more gently!" is displayed on the upper part of the user-obtained image as shown in Fig. 2 on image displayer 11. In the case that the area of blue pattern 35 of user-obtained image 31 is smaller than blue pattern 36 of model image 32, an attention message regarding compression such as "Please press a little harder!" is displayed on the upper part of the user-obtained image on image displayer 11.

In part of Fig. 2 (2), it is set so that the graph of pressure/strain data during operation is displayed along with time passage, based on the pressure information from the pressure sensor mounted in probe 2 and the strain data from strain calculating unit 6. As for the example of displaying graphs, there are styles such as the scroll-style or the bar-graph style as shown in Fig. 2. To this graph, the letters to indicate the threshold value of "insufficient compression/strain" are created by graphic unit 8 and displayed when there is a shortage of pressure, and the letters to indicate the threshold value of "the excessive compression/strain" is created by graphic unit 8 and displayed when there is excessive pressure.

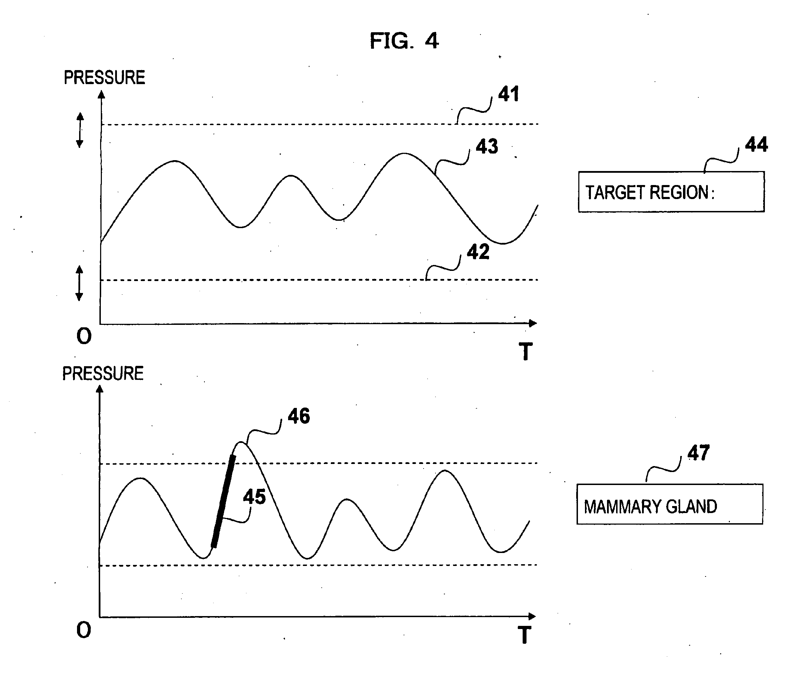

The details of this graph will now be described referring to Fig. 4. Upper limit value 41 as the threshold value of "excessive compression/strain", lower limit value 42 as the threshold value of "insufficient compression/strain", and curve graph 43 indicating the pressure/strain are displayed in time series. When curve graph 43 is at value 46 being outside of the proper threshold regarding pressure at the moment, control calculating unit 12 notifies the user by displaying a blinking light, sonant (sound such as beep-sound voice) or warning message.

Also, conceivable factors as improper compression are cases such as the time when the pressure (strain) is above (below) the threshold value, the time when the change of pressure is extremely larger (smaller) due to pressure/ΔT, or when strain rate is high. For example, in the case of being subjected to sudden compression as seen in heavy line graph 45, the strain cannot be measured accurately since the pressure against hard portion in the training phantom or subject 1 glide to a different direction from the direction of compression. Therefore, when the gradient of the graph is more than, for example, 60 degrees or the strain rate is more than 1.7 being abnormal, control calculating unit 12 determines that drastic compression is being subjected, and indicates that to the user by displaying a blinking, sonant (a beep sound or voice) or a warning message. Moreover, since the hardness varies depending on the measurement portion such as a mammary gland or prostate gland, the most suitable degree of compression for the measurement portion also varies. Given this factor, control calculating unit 12 varies upper limit value 41 for indicating "excessive compression/strain" and lower limit value 42 indicating "insufficient compression/strain" when a certain measurement portion is inputted to display box 44 using operation unit 13. For example, in the case for mammary gland, since the region surrounding the measurement portion has soft structure, control-calculating unit 12 lowers both upper limit value 41 and lower limit value 42, and broadens the distance between the upper limit value 41 and lower limit value 42. In the case of the prostate gland, since the region surrounding the measurement portion has hard structure, control calculating unit 12 raises both upper limit value 41 and lower limit value 42, and narrows down the distance between upper limit value 41 and lower limit value 42.

Also, control calculating unit 12 may set upper limit value 41 and lower limit value 42 according to the kind of probe 2. Probe 2 may be, for example, a convex probe, radial probe or sector probe, each having a different degree of most suitable compression. For example, the convex probe is mainly for measuring the mammary gland, thus control-calculating unit 12 lowers both upper limit value 41 and lower limit value 42 and broadens the distance between upper limit value 41 and lower limit value 42 to make the stress to be 20Kpa. In the radial probe, control-calculating unit 12 raises both upper limit value 41 and lower limit value 42 and narrows down the distance between upper limit value 41 and lower limit value 42 to make the stress to be 60Kpa. The sector probe is for observing a pancreas, aorta or vascular channel, thus the upper value 41 and lower value 42 are both set on the initial value (default state) to make the stress to be 40Kpa.

Fig. 2 (3) is a display of information on the present direction or dispersion state of compression made by probe 2 obtained by control calculation unit 12, in order to help users to recognize them. More specifically, as shown in the diagram, whether the compression direction of probe 2 is appropriate or not is displayed on image displayer 11 by indicating the direction and the measurement of the force by the direction and measurement of the arrows. In this embodiment, a plurality of arrows is indicated since the plurality of pressure sensors are used. By these arrows, the user can view the compression direction and measurement of the plurality of pressure sensors, and check the bias of compression on the screen. In addition, a warning message or attention message such as "Please push L-side a little harder!" is displayed under the arrows in Fig. 2.

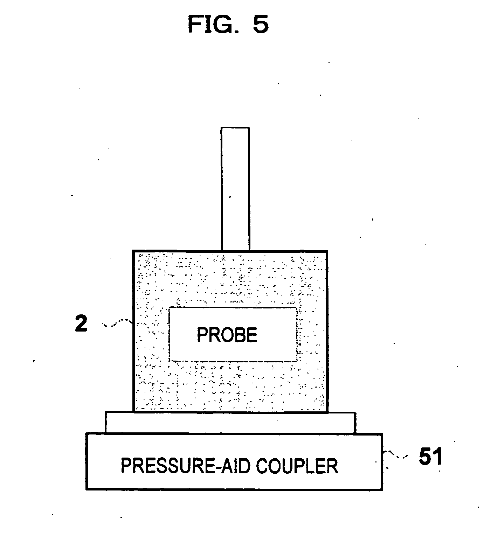

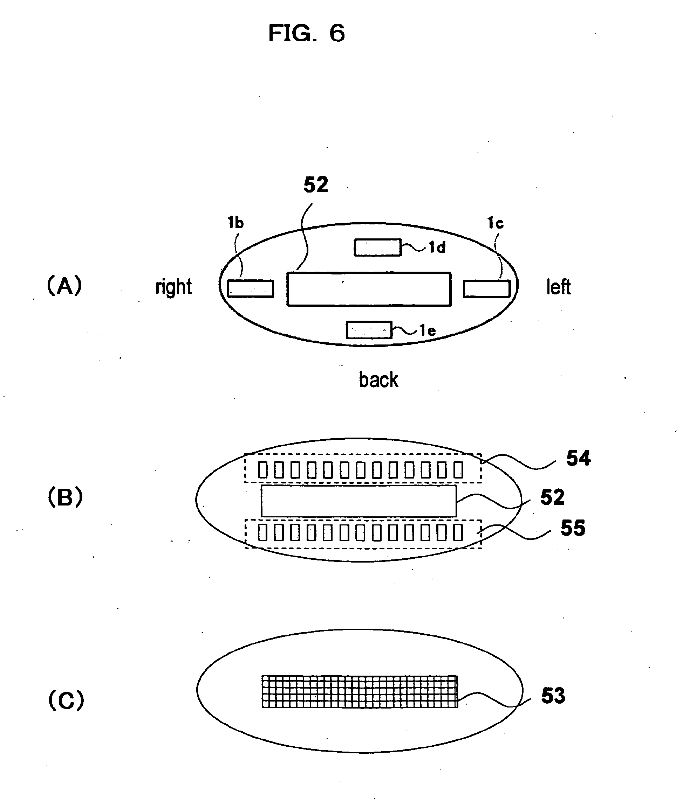

The configuration of the pressure sensor of probe 2 will now be described referring to Figs. 5 and 6. Fig. 6 (A) is a diagram showing the first configuration of a probe using a plurality of pressure sensors. Pressure sensors 1b, 1c, 1d and 1e are disposed in four corners, right side, left side, front side and back side at the tip of pressure-aid coupler 51 of probe 2. Control calculating unit 12 displays information of the pressure dispersion as shown in Fig. 2 (3) using marks such as arrows, based on the pressure information from previously mentioned four pressure sensors 1b, 1c, 1d and 1e. On the condition that any one direction of pressure is lacking, the shortage of pressure in that region is indicated by blinking or displaying the direction, that makes it possible for the operator to add more pressure in the indicated direction. This enables an ideal scanning of the image by providing pressure evenly on the pressed surface.

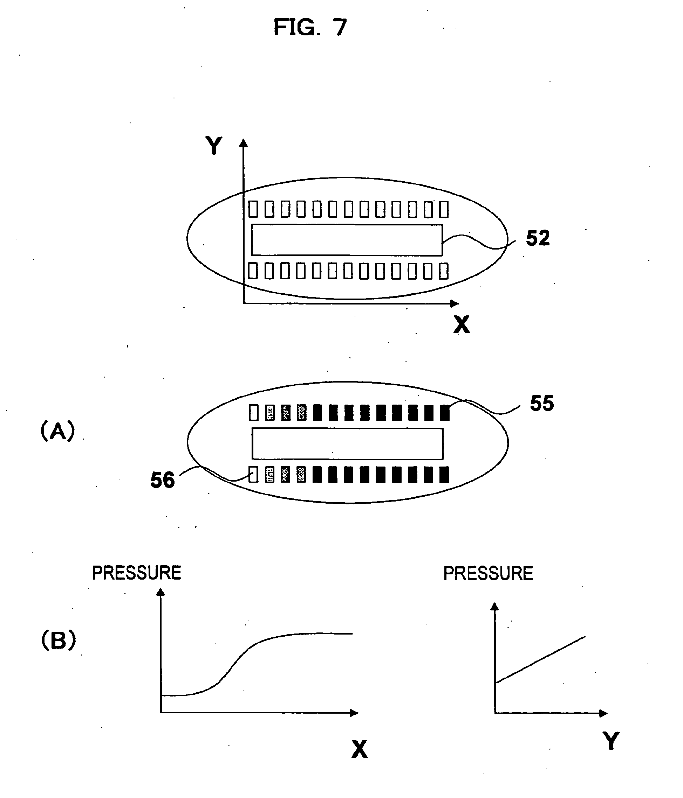

Fig. 6 (B) is a diagram showing the second configuration of the probe in Fig. 2 using a plurality of pressure sensors. At the tip of pressure-aid coupler 51 of probe 2, a plurality of pressure sensors are arrayed along the long-axis direction on the opposite sides of transducer 52, which forms upper-side pressure sensor group 54 and under-side pressure sensor group 55. Control calculating unit 12 displays information on the pressure dispersion as shown in Fig. 7 based on the pressure information from the pressure sensors arrayed in two rows.

As shown in Fig. 7 (A), pressure information is displayed in contrasting density or in color on image displayer 11. The pressure display in Fig. 7 (A) is displayed corresponding to the position of the pressure sensors of transducer 52 and pressure-aid coupler 51. When pressure-aid coupler 51 is pressed against the training phantom or subject 1 with heavy pressure, pressure information image 55 is displayed densely. On the contrary, when pressure-aid coupler 51 is pressed against the training phantom or subject 1 with light pressure, pressure information image 56 is displayed in thin contrast density. Since the right side of the transducer is displayed densely, the user can recognize that the pressure is concentrated on the right side of probe 2 as being applied. Thus the user adjusts probe 2 by giving more pressure on the left side as being applied to the subject. Since these pressure sensors are arrayed at least in two rows, the user can also grasp the pressure condition of upper and lower parts of the probe.

Moreover, the pressure information can be broken down into X and Y directions and displayed on a graph as shown in Fig. 7 (B). The major-axis direction of pressure-aid coupler 51 is set as X-axis direction and the minor-axis direction is set as Y-axis direction. It means that the pressure distribution applied on the X-direction and the pressure distribution applied on the Y-direction are displayed in a graph. For example, if the right side of the X-axis graph is displayed larger, it means that the pressure is concentrated on the right side as applying probe 2. Therefore, the user adjusts the pressure to be applied more on the left side of probe 2. In the Y-axis graph, if the upper side is indicated larger, it means that the pressure is concentrated on the upper side as applying probe 2. Therefore, the user adjusts the pressure to be applied more on the lower side of probe 2.

Fig. 6 (C) is a diagram showing the third configuration of the probe using a plurality of pressure sensors in Fig. 2. A transducer of probe 2 is comprised by a plurality of cMUT elements 53, and these cMUT elements 53 are used to measure the pressure. Concretely, when pressure is applied on cMUT elements 53 being used as a capacitance-type pressure sensor, the insulating diaphragm membrane are distorted by the pressure, and the distance between the movable electrode and the fixed electrode is changed by the distortion of the diaphragm membrane. And electric capacitance of the capacitor including the movable electrode and the fixed electrode changes based on the change of the previously mentioned distance. The pressure is detected by detecting the change of the distance. The pressure information as shown in Fig. 7 can be displayed on image displayer 11 using probe 2 using cMUT elements 53.

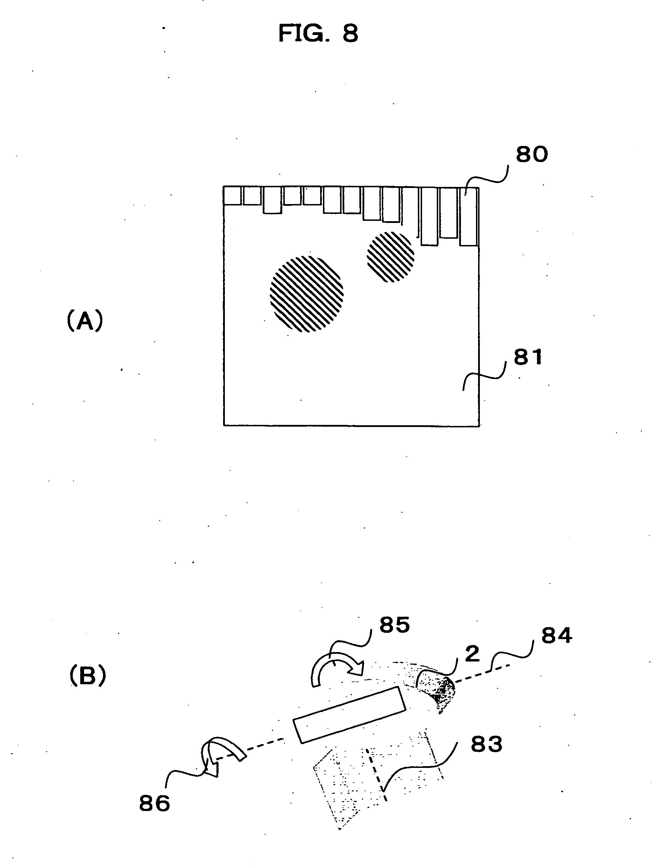

Next, an example for displaying pressure graph 80 is illustrated in Fig. 8 (A). Control calculating unit 12 makes user-obtained image 81 to display pressure graph 80. In the cases that a plurality of pressure sensors are arrayed in pressure-aid coupler 51 of probe 2 or probe 2 is configured by cMUT elements 53, the pressure information is displayed on an image at the position to which the pressure sensor corresponds. In concrete terms, pressure graph 80 is displayed on the upper edge part of user-obtained image 81 at the image display position corresponding to the pressure sensor.

In an example of Fig. 8(A), the fact can be recognized that the pressure is applied more on the right side of user-obtained image 81. It means that the pressure is applied more on the left side of probe 2, thus the user adjusts the pressure to be applied more on the right side as applying to the subject. In this way, the pressure information can be obtained while observing the screen by displaying pressure graph 80 on user-obtained image 81, which improves the efficiency of diagnosis.
In addition, pressure graph 80 can be translucent so that it will not have much effect on user-obtained image 81. Also, pressure graph 80 may be displayed in colors, for example, the heavy pressure may be displayed in yellow and the light pressure in white.

Also, the configuration to induce the application direction of probe 2 by indicating the tilt direction of the probe on the screen to the user will be illustrated in Fig. 8 (B). Control calculating unit 12 figures out the pressure distribution applied on major axis 84 (X-axis) direction and the pressure distribution applied on minor axis 83 (Y-axis) direction, calculates and displays how much probe 2 needs to be tilted. For example, if the pressure is heavier on the left side in major axis 84 (X-axis), indication arrow 85 is displayed to rotate in a clockwise direction. If the pressure is heavier on the upper side in minor axis 83 (Y-axis), indication arrow 85 is displayed to rotate downward. The degree of the tilt is displayed in real time by the length or thickness of the arrows.

In this way, indication arrow 86 for inducing the rotation centering around major axis 84 (X-axis) of probe 2 and indication arrow 85 for inducing the rotation centering around minor axis 83 (Y-axis) of probe 2 are displayed. Then the instruction on the degree to tilt the probe from the present application state with respect to the training phantom or subject 1 is provided. According to the instruction of indication arrow 85 and indication arrow 86, the user adjusts the pressure to be properly added as applying probe 2.

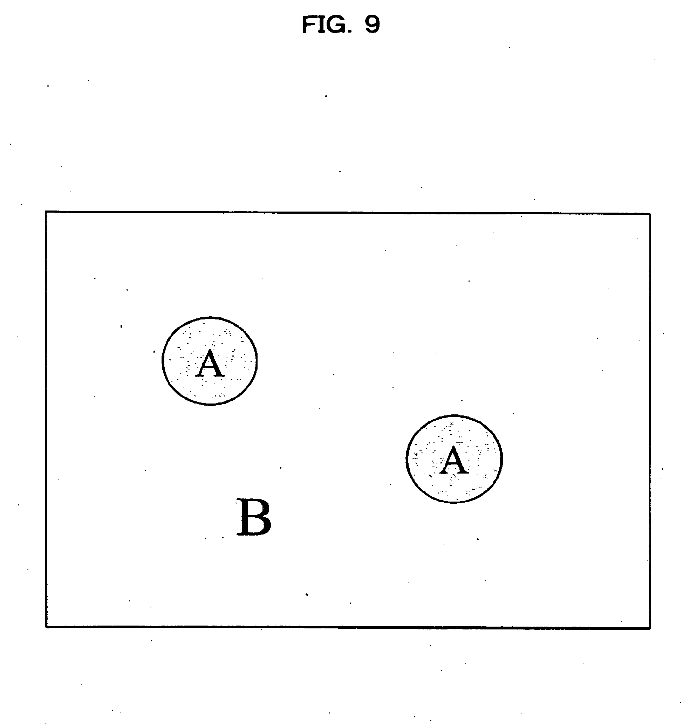

Fig. 9 is a diagram showing an example of the training phantom simulating a living body. There are training phantoms for different organs such as, for example, a mammary gland, prostate gland, thyroid gland or liver. In Fig. 9, shaded parts "A" indicate elasticity module, reflectance ratio and figure of the tissues of interest such as cancer being simulated, and part "B" indicates elasticity module, reflectance ratio and figure of normal tissues such as fat (breast tissues for a mammary gland) being simulated. With the use of such training phantom simulating the living body, the user is able to carry out the training as if depicting the tissue elasticity module image of a real living body, and to carry out with the training more effectively. The guiding of the pressure according to the training phantom becomes possible by controlling such information of the training phantom by the controlling unit using information such as ID.

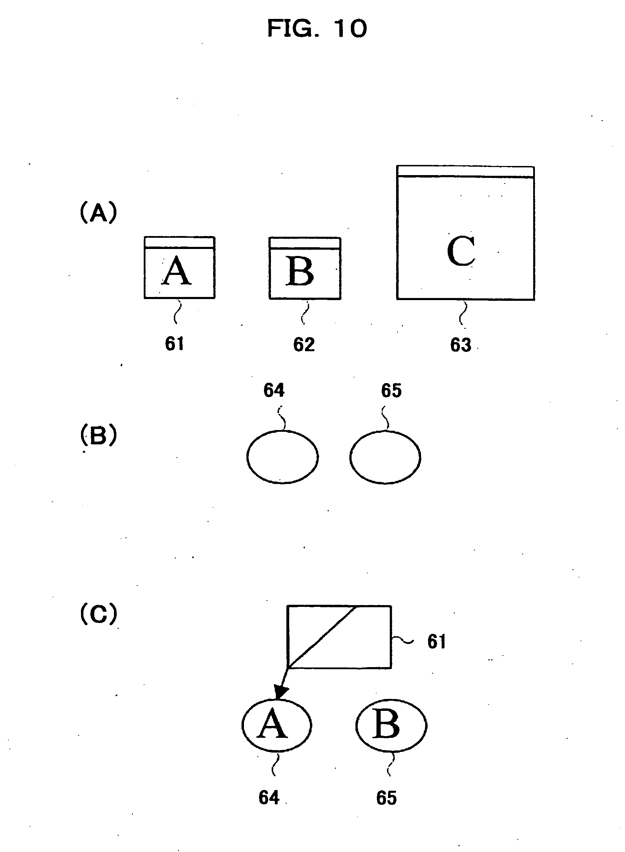

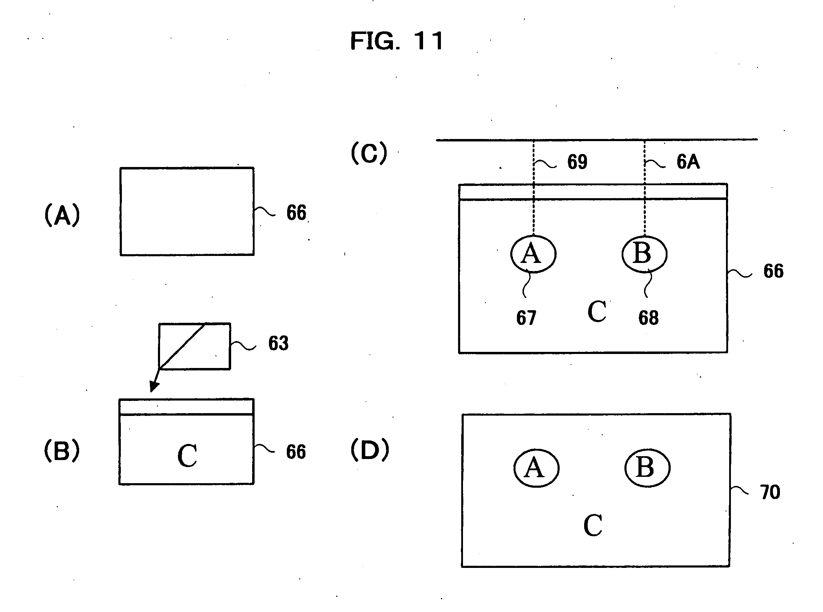

Figs. 10 and 11 are diagrams showing an example of the method for manufacturing the training phantom in Fig. 9. Here, a method for manufacturing the training phantom simulating a living body using vegetable gelatin.
First, liquid solutions A, B and C are prepared in beakers 61 - 63 as shown in Fig. 10 (A). Ultrasonic scatterer is put into each of the liquid solution, and liquid solution A and liquid solution B is made to have the same consistency (luminance), and are stirred thoroughly as being deaerated. In this case, the relationship between the liquid solutions is as follows:
A: the consistency of the vegetable gelatin is high (hard)
B: the consistency of the vegetable gelatin is low (soft)
C: the consistency of the vegetable gelatin is low (soft)

Next, cases 64 and 65 respectively simulating a malignant tumor (cancer) and benign tumor are prepared as shown in Fig. 10 (B). Case 64 is for a malignant tumor (cancer), and case 65 is for a, benign tumor. As shown in Fig. 10 (C), liquid solution A for hard tissues is poured in case 64, and liquid solution B for soft tissues is poured in case 65, both to be cooled and coagulated. Vegetable gelatin can be coagulated and stored at room temperature without problems, since its melting point is 45 degrees C and the boiling point is 80 degrees C. When liquid solutions A and B are coagulated, they are to be taken out of cases 64 and 65.

Next, phantom case 66 for the surrounding tissues as shown in Fig. 11 (B) is prepared. Liquid solution C for the surrounding tissues is poured into this phantom case 66 as shown in Fig. 11 (B). The coagulated substances 67 and 68 taken out of cases 64 and 65 are suspended therein using strings 69 and 6A, and immobilized in liquid solution C in phantom case 66. The vegetable gelatin is coagulated through storing it at room temperature in the above-mentioned condition, thus training phantom 70 as seen in Fig. 11 (D) is completed when the coagulated gelatin is taken out of phantom case 66. It is desirable that the wrapping process is implemented on training phantom 70 after being taken out.

## Claims

1. An ultrasound diagnostic apparatus comprising:
an ultrasonic probe (2),
an ultrasound transmitting/receiving unit (3) adapted to cause the ultrasonic probe to transmit/receive ultrasonic waves to and from a subject or phantom,
signal processing means (6, 7) adapted to process signals detected by the ultrasonic probe,
pressure detecting means (1, 53 to 54) adapted to detect the pressure at a plurality of positions between the ultrasonic probe and the subject or phantom, and
display means (10, 11) adapted to display a strain elasticity image, and to display a pressure condition at said positions
**characterized in that** the display means (10, 11) is adapted to display the strain elasticity image created in real time by the signal processing means (6, 7), in parallel with a strain elasticity image imaged in advance as a model image.

2. The ultrasound diagnostic apparatus according to claim 1, wherein the ultrasonic probe (2) comprises a pressure-aid coupler (51) in which a plurality of said pressure detecting means (1, 53-55) are arranged.

3. The ultrasound diagnostic apparatus according to claim 1, wherein the display means (10, 11) is adapted, when any direction of the plurality of pressure detecting means (1, 53-55) lacks pressure, to display which direction has insufficient pressure.

4. The ultrasound diagnostic apparatus according to claim 1, wherein the display means (10, 11) is adapted to display the pressure information corresponding to the arrangement of the pressure detecting means (1, 53-55) by image contrasting density or using an arrow.

5. The ultrasound diagnostic apparatus according to claim 1, wherein the display means (10, 11) is adapted to display the pressure information corresponding to the arrangement of the pressure detecting means (1, 53-55) in graph form.

6. The ultrasound diagnostic apparatus according to claim 1, wherein the pressure detecting means (1, 53-55) are cMUT elements for detecting pressure by the change of electric capacitance.

7. The ultrasound diagnostic apparatus according to claim 1, wherein the display means (10, 11) is adapted to display a pressure graph at the position corresponding to the pressure detecting means (1, 53-55) on the strain elasticity image.

8. The ultrasound diagnostic apparatus according to claim 1, wherein indication arrows for rotating the probe centering around the major axis and the minor axis of the probe (2) are respectively displayed based on the pressure condition of the probe.

9. The ultrasound diagnostic apparatus according to claim 1, wherein the display means (10, 11) is adapted to display the strain amount and/or the pressure obtained upon construction of the strain elasticity image in graph form.

10. The ultrasound diagnostic apparatus according to claim 1, comprising a table identifying a compression method for each target region of the subject or phantom, and adapted to display the compression method corresponding to the target region.

11. The ultrasound diagnostic apparatus according to claim 1, comprising detecting means (12) for superimposing the strain elasticity image obtained in real time and the strain elasticity model image imaged in advance, and detecting the coincidence between the strain elasticity image obtained in real time and the strain elasticity model image.

12. The ultrasound diagnostic apparatus according to claim 11, wherein the detecting means (12) is adapted to binarize and superimpose hue information of the strain elasticity image obtained in real time and the strain elasticity model image imaged in advance.

13. The ultrasound diagnostic apparatus according to claim 11, wherein the detecting means (12) is adapted to detect the coincidence of the superimposed strain elasticity image obtained in real time and the strain elasticity model image imaged in advance, by the area or pixels of the parts that do not overlap.

14. The ultrasound diagnostic apparatus according to claim 11, wherein the display means (10, 11) is adapted to display an attention message in compliance with the coincidence.

15. The ultrasound diagnostic apparatus according to claim 1, wherein the display means (10, 11) is adapted to display the obtained pressure information in graph form with passage of time.

## Patentansprüche

1. Ultraschall-Diagnosevorrichtung aufweisend:
eine Ultraschallsonde (2),
eine Ultraschallsende-/Empfangseinheit (3), um die Ultraschallsonde zu veranlassen, Ultraschallwellen an ein Subjekt oder Phantom zu senden bzw. davon zu empfangen,
eine Signalverarbeitungseinrichtung (6, 7) zum Verarbeiten von von der Ultraschallsonde erfaßten Signalen,
Druckerfassungseinrichtungen (1, 53 bis 54) zum Erfassen des Drucks an mehreren Orten zwischen der Ultraschallsonde und dem Subjekt oder Phantom, und
eine Anzeigeeinrichtung (10, 11) zum Anzeigen eines Spannungs-Elastizitätsbilds und zur Anzeige eines Druckzustands an den genannten Orten,
**dadurch gekennzeichnet, daß** die Anzeigeeinrichtung (10, 11) eingerichtet ist, das Spannungs-Elastizitätsbild, das von der Signalverarbeitungseinrichtung (6, 7) in Echtzeit erzeugt wird, parallel zu einem vorab als Modellbild aufgenommenen Spannungs-Elastizitätsbild anzuzeigen.

2. Vorrichtung nach Anspruch 1, wobei die Ultraschallsonde (2) einen Drückhilfekoppler (51) aufweist, in dem mehrere der Druckerfassungseinrichtungen (1, 53 bis 55) angeordnet sind.

3. Vorrichtung nach Anspruch 1, wobei die Anzeigeeinrichtung (10, 11) eingerichtet ist dann, wenn irgendeiner Seite der Druckerfassungseinrichtungen (1, 53 bis 55) Druck fehlt, anzuzeigen, welche Seite ungenügenden Druck aufweist.

4. Vorrichtung nach Anspruch 1, wobei die Anzeigeeinrichtung (10, 11) eingerichtet ist, die Druckinformationen entsprechend der Anordnung der Druckerfassungseinrichtungen (1, 53 bis 55) mittels einer Bildkontrastdichte oder unter Verwendung eines Pfeils anzuzeigen.

5. Vorrichtung nach Anspruch 1, wobei die Anzeigeeinrichtung (10, 11) eingerichtet ist, die Druckinformationen entsprechend der Anordnung der Druckerfassungseinrichtungen (1, 53 bis 55) in Form eines Graphen anzuzeigen.

6. Vorrichtung nach Anspruch 1, wobei die Druckerfassungseinrichtungen (1, 53 bis 55) cMUT Elemente zur Erfassung von Druck durch die Änderung elektrischer Kapazität sind.

7. Vorrichtung nach Anspruch 1, wobei die Anzeigeeinrichtung (10, 11) eingerichtet ist, am jeweils den Druckerfassungseinrichtungen (1, 53 bis 55) entsprechenden Ort auf dem Spannungs-Elastizitätsbild einen Druckgraphen anzuzeigen.

8. Vorrichtung nach Anspruch 1, wobei aufgrund des Druckzustands der Sonde jeweils Anzeigepfeile zum Drehen der Sonde (2) zentriert um deren Haupt- und Nebenachse angezeigt werden.

9. Vorrichtung nach Anspruch 1, wobei die Anzeigeeinrichtung (10, 11) eingerichtet ist, den bei Aufbau des Spannungs-Elastizitätsbilds gewonnenen Druck und/oder Spannungswert in Form eines Graphen anzuzeigen.

10. Vorrichtung nach Anspruch 1, mit einer Tabelle, die einen Kompressionsvorgang für jeden Zielbereich des Subjekts oder Phantoms angibt, wobei die Vorrichtung zum Anzeigen des Kompressionsvorgangs entsprechend dem Zielbereich eingerichtet ist.

11. Vorrichtung nach Anspruch 1, mit einer Erfassungseinrichtung (12) zum Überlagern des in Echtzeit erhaltenen Spannungs-Elastizitätsbilds und des vorab aufgenommenen Spannungs-Elstizitäts-Modellbilds und zum Erfassen eines Zusammenfallens zwischen dem in Echtzeit gewonnenen Spannungs-Elastizitätsbild und dem Spannungs-Elastizitäts-Modellbild.

12. Vorrichtung nach Anspruch 11, wobei die Erfassungseinrichtung (12) eingerichtet ist, Tönungsinformationen des in Echtzeit gewonnenen Spannungs-Elastizitätsbilds und des vorab aufgenommenen Spannungs-Elastizitäts-Modellbilds zu digitalisieren und einander zu überlagern.

13. Vorrichtung nach Anspruch 11, wobei die Erfassungseinrichtung (12) eingerichtet ist, das Zusammenfallen des in Echtzeit gewonnenen Spannungs-Elastizitätsbilds und des vorab aufgenommenen Spannungs-Elastizitäts-Modellbilds, die überlagert sind, durch die Fläche oder die Pixel der einander nicht überlappenden Teile zu erfassen.

14. Vorrichtung nach Anspruch 11, wobei die Anzeigeeinrichtung (10, 11) eingerichtet ist, entsprechend dem Zusammenfallen eine Warnmeldung anzuzeigen.

15. Vorrichtung nach Anspruch 1, wobei die Anzeigeeinrichtung (10, 11) eingerichtet ist, die gewonnenen Druckinformationen entsprechend dem zeitlichen Ablauf in Graphenform anzuzeigen.

## Revendications

1. Appareil de diagnostic par ultrasons, comprenant :
une sonde à ultrasons (2),
une unité d'émission/réception d'ultrasons (3) adaptée pour permettre à la sonde à ultrasons d'émettre/recevoir des ondes ultrasonores vers et depuis un sujet ou mannequin,
un moyen de traitement de signal (6, 7) adapté pour traiter des signaux détectés par la sonde à ultrasons,
des moyens de détection de pression (1, 53 à 54) adaptés pour détecter la pression en une pluralité de positions entre la sonde à ultrasons et le sujet ou mannequin, et
un moyen d'affichage (10,11) adapté pour afficher une image d'élasticité ou déformation, et pour afficher un état de pression au niveau desdites positions,
**caractérisé en ce que** le moyen d'affichage (10, 11) est adapté pour afficher l'image d'élasticité ou déformation créée en temps réel par le moyen de traitement de signal (6, 7), en parallèle avec l'image d'élasticité ou déformation affichée à l'avance sous forme d'une image modèle.

2. Appareil de diagnostic par ultrasons selon la revendication 1, dans lequel la sonde à ultrasons (2) comprend un coupleur d'aide à la pression (51) dans lequel une pluralité desdits moyens de détection de pression (1, 53 - 55) est disposée.

3. Appareil de diagnostic par ultrasons selon la revendication 1, dans lequel le moyen d'affichage (10, 11) est adapté, quand une quelconque direction de la pluralité de moyens de détection de pression (1, 53 - 55) manque de pression, pour afficher la direction qui a une pression insuffisante.

4. Appareil de diagnostic par ultrasons selon la revendication 1, dans lequel le moyen d'affichage (10,11) est adapté pour afficher l'information de pression correspondant à la disposition des moyens de détection de pression (1, 53 - 55) par densité de contraste d'image ou à l'aide d'une flèche.

5. Appareil de diagnostic par ultrasons selon la revendication 1, dans lequel le moyen d'affichage (10,11) est adapté pour afficher l'information de pression correspondant à la disposition des moyens de détection de pression (1, 53 - 55) sous forme de graphique.

6. Appareil de diagnostic par ultrasons selon la revendication 1, dans lequel les moyens de détection de pression (1, 53 - 55) sont des éléments cMUT (capteur ultrasonique à effet capacitif) pour détecter une pression par le changement de capacité électrique.

7. Appareil de diagnostic par ultrasons selon la revendication 1, dans lequel le moyen d'affichage (10,11) est adapté pour afficher un graphique de pression au niveau de la position correspondant aux moyens de détection de pression (1, 53 - 55) sur l'image d'élasticité ou déformation.

8. Appareil de diagnostic par ultrasons selon la revendication 1, dans lequel des flèches indicatrices pour faire tourner la sonde autour du grand axe et du petit axe de la sonde (2) sont affichées respectivement en fonction de l'état de pression de la sonde.

9. Appareil de diagnostic par ultrasons selon la revendication 1, dans lequel le moyen d'affichage (10, 11) est adapté pour afficher la quantité de déformation et/ou la pression obtenue lors de la construction de l'image d'élasticité ou déformation sous forme de graphique.

10. Appareil de diagnostic par ultrasons selon la revendication 1, comprenant un tableau identifiant une méthode de compression pour chaque région cible du sujet ou mannequin, et adapté pour afficher la méthode de compression correspondant à la région cible.

11. Appareil de diagnostic par ultrasons selon la revendication 1, comprenant un moyen de détection (12) pour superposer l'image d'élasticité ou déformation obtenue en temps réel et l'image modèle d'élasticité ou déformation affichée à l'avance, et pour détecter la coïncidence entre l'image d'élasticité ou déformation obtenue en temps réel et l'image modèle d'élasticité ou déformation.

12. Appareil de diagnostic par ultrasons selon la revendication 11, dans lequel le moyen de détection (12) est adapté pour binariser et superposer une information de teinte de l'image d'élasticité ou déformation obtenue en temps réel et de l'image modèle d'élasticité ou déformation affichée à l'avance.

13. Appareil de diagnostic par ultrasons selon la revendication 11, dans lequel le moyen de détection (12) est adapté pour détecter la coïncidence de l'image d'élasticité ou déformation superposée obtenue en temps réel et de l'image modèle d'élasticité ou déformation affichée à l'avance, par la surface ou les pixels des parties qui ne se chevauchent pas.

14. Appareil de diagnostic par ultrasons selon la revendication 11, dans lequel le moyen d'affichage (10, 11) est adapté pour afficher un message d'avertissement en fonction de la coïncidence.

15. Appareil de diagnostic par ultrasons selon la revendication 1, dans lequel le moyen d'affichage (10,11) est adapté pour afficher l'information de pression obtenue sous forme de graphique à mesure que le temps passe.
